# EUROPÄISCHE PATENTSCHRIFT

(11) **EP 4 036 558 B1**
(45) Veröffentlichungstag und Bekanntmachung des Hinweises auf die Patenterteilung: **24.07.2024**
(21) Anmeldenummer: 21154707.0
(22) Anmeldetag: 02.02.2021
(51) Int. Cl.: G01N 21/47, G01N 21/51, G01N 21/53, G01N 21/85, G01N 33/18

(54) **STREULICHTSENSOR UND VERFAHREN ZU SEINER HERSTELLUNG**
SCATTERED LIGHT SENSOR AND METHOD FOR MANUFACTURING IT
CAPTEUR DE LUMIÈRE DIFFUSE ET PROCÉDÉ DE SA FABRICATION

(43) Veröffentlichungstag der Anmeldung: 03.08.2022
(73) Patentinhaber: Exner & Tottewitz Besitz GBR, 76275 Ettlingen (DE)
(72) Erfinder: EXNER, Detlef, 71297 Mönsheim (DE); TOTTEWITZ, Michael, 76646 Bruchsal (DE)
(74) Vertreter: LBP Lemcke, Brommer & Partner Patentanwälte mbB

(56) Entgegenhaltungen:
- DE-A1- 102013 103 735
- US-A- 5 350 922

## Beschreibung

Die Erfindung betrifft einen Streulichtsensor zur Trübungsmessungen und ein Verfahren zur Herstellung eines Streulichtsensors zur Trübungsmessung. Streulichtsensoren werden zur Überwachung und Steuerung von Produktionsprozessen, Umweltprozessen und Reinigungsprozessen eingesetzt. Bei Streulichtsensoren wird mittels einer Strahlungsquelle ein Messstrahl in das zu messende Medium ausgesandt und mittels eines Detektors wird der reflektierte oder gestreute Anteil des Messstrahls als Empfangsstrahl detektiert. Bei typischen Anwendungen ist es wünschenswert, den Streulichtsensor über ein Zuführrohr oder eine Zuführöffnung in ein Behältnis oder eine Leitung mit dem zu messenden Medium einzuführen. Hierdurch ergeben sich hohe Anforderungen an eine kompakte Bauform, um den notwendigen Durchmesser des Zuführrohres oder der Öffnung für den Streulichtsensor gering zu halten.

Aus DE 10 2017 107 963 A1 ist ein Rückstreusensor bekannt, bei welchem mittels einer bikonvexen Linse ein optisches Abstimmelement ausgebildet wird, um einen Kontakt zwischen dem zu messenden Medium und Detektor sowie Strahlungsquelle zu vermeiden.

Weiter ist aus der DE 10 2013 103 735 A1 ein Trübungssensor mit einem länglichen Sensorgehäuse, einer Strahlungsquelle und einem Strahlungsdetektor bekannt, wobei das Sensorgehäuse einen seitlichen Austrittsfensterbereich für den Austritt eines Messstrahls und einen seitlichen Eintrittsfensterbereich für den Eintritt eines Empfangsstrahls aufweist.

Die US 5250922 A1 schlägt einen Streulichtsensor mit einem zylindrischen Sensorgehäuse, einer Lichtquelle und einem Streulichtdetektor vor. Das Sensorgehäuse weist einen seitlichen Austrittsfensterbereich für den Austritt eines Messstrahls und einen seitlichen Eintrittsfensterbereich für den Eintritt eines Streulichtstrahls auf. Die Lichtquelle und der Streulichtdetektor sind in einem Halterungselement angeordnet, das einen Lichtstopp zwischen der Lichtquelle und dem Streulichtdetektor aufweist, um zu vermeiden, dass Licht von der Lichtquelle direkt zum Streulichtdetektor gelangt.

Der vorliegenden Erfindung liegt die Aufgabe zugrunde, einen Streulichtsensor sowie ein Verfahren zu seiner Herstellung zur Verfügung zu stellen, welches eine kompaktere und dennoch robuste Ausgestaltung des Streulichtsensors ermöglicht.

Gelöst ist diese Aufgabe durch einen Streulichtsensor gemäß Anspruch 1 sowie ein Verfahren seiner Herstellung gemäß Anspruch 13. Vorteilhafte Ausgestaltungen finden sich in den abhängigen Ansprüchen.

Der erfindungsgemäße Streulichtsensor ist zur Trübungsmessung in einem flüssigen, pastösen, pulvrigen und/oder gasförmigen Medium geeignet.

Der Streulichtsensor weist eine Strahlungsquelle zum Erzeugen eines Messstrahls, einen Detektor zum Detektieren des in dem Medium zumindest teilweise reflektierten und/oder gestreuten Messstrahls als Empfangsstrahl und ein längliches Sensorgehäuse zum Eintauchen in das Medium auf. Strahlungsquelle und Detektor sind in dem Sensorgehäuse angeordnet. Der Streulichtsensor weist ein Befestigungsmittel zum Anordnen des Sensorgehäuses an einer Behälterwand auf.

Wesentlich ist, dass in einer seitlichen Wand des Sensorgehäuses zwischen Befestigungsmittel und einem Endbereich des Sensorgehäuses zumindest ein Austrittsfensterbereich für den Austritt des Messstrahls aus dem Sensorgehäuse und ein Eintrittsfensterbereich für den Eintritt des Empfangsstrahls ausgebildet ist, dass der Streulichtsensor ein in das Sensorgehäuse angeordnetes Halterungselement aufweist, an welchem Strahlungsquelle und Detektor derart angeordnet sind, dass eine optische Achse der Strahlungsquelle mit einer optischen Achse des Detektors einen Winkel im Bereich 50° und 130° einschließt und dass das Halterungselement als optisches Abschattungselement für die Messstrahlung ausgebildet ist, sodass der Detektor gegenüber direkten Strahlengängen zwischen Strahlungsquelle und Detektor innerhalb des Sensorgehäuses abgeschattet ist. Weiterhin ist wesentlich, dass zwischen Detektor und Strahlungsquelle eine um das Halterungselement umlaufende Abschirmung innerhalb des Sensorgehäuses angeordnet ist.

Die Erfindung ist in der Erkenntnis begründet, dass ein seitlicher Austritt des Messstrahls und ein seitlicher Eintritt des Empfangsstrahls in das Sensorgehäuse eine kompaktere Bauform ermöglicht. Während bei vorbekannten Streulichtsensoren typischerweise die optische Achse von Detektor und/oder Strahlungsquelle parallel zu der Längsachse des länglich ausgebildeten Sensorgehäuses angeordnet sind, kann bei dem erfindungsgemäßen Rückstreusensor eine schräge Anordnung relativ zur Längsachse des Sensorgehäuses bei Strahlungsquelle und Detektor erfolgen. Insbesondere können Strahlungsquelle und Detektor in Richtung der Längsachse des Sensorgehäuses hintereinander angeordnet werden, sodass ein Sensorgehäuse mit geringerer Querschnittsfläche, insbesondere mit einem geringeren Durchmesser, ausgebildet werden kann.

Bei kompakten Bauformen zeigten Untersuchungen der Anmelder jedoch, dass die Ausbildung eines robusten Streulichtsensors, der insbesondere auch gegenüber Erschütterungen unempfindlich ist, eine besondere Herausforderung darstellt. Der erfindungsgemäße Streulichtsensor weist ein Halterungselement auf, an welchem Strahlungsquelle und Detektor derart angeordnet sind, dass eine optische Achse der Strahlungsquelle mit einer optischen Achse des Detektors ein Winkel im Bereich 50° bis 130° einschließt. Hierdurch kann bei Herstellung des Streulichtsensors zunächst die Anordnung von Strahlungsquelle und Detektor an dem Halterungselement erfolgen und anschließend das Halterungselement mit Detektor und Strahlungsquelle in dem Sensorgehäuse angeordnet werden. Dies ermöglicht einen kompakten und zugleich robusten Aufbau.

Darüber hinaus ist das Halterungselement als optisches Abschattungselement für die Messstrahlung ausgebildet, sodass der Detektor gegenüber direkten (geradlinigen) Strahlengängen zwischen Strahlungsquelle und Detektor innerhalb des Sensorgehäuses durch das Halterungselement abgeschattet ist. Hierdurch wird ein kompakter Aufbau begünstigt, da bei Anordnen von Strahlungsquelle und Detektor direktes Streulicht innerhalb des Sensorgehäuses nicht berücksichtigt werden muss.

Vorteilhafterweise sind der Austrittsfensterbereich und der Eintrittsfensterbereich parallel zu einer Längserstreckung des länglichen Sensorgehäuses angeordnet. Hierdurch kann ein konstruktiv einfacher Aufbau erzielt werden, da dennoch Vor- und Rücksprünge an der Außenwand des Sensorgehäuses im Bereich von Austrittsfensterbereich und Eintrittsfensterbereich vermieden werden können, sodass das Risiko einer Schmutzablagerung verringert wird.

Wie vorangehend erwähnt, ist das Sensorgehäuse des erfindungsgemäßen Streulichtsensors länglich ausgebildet. Ein das Sensorgehäuse einhüllender Kreiszylinder weist somit ein Aspektverhältnis von Länge zu Durchmesser größer 1, bevorzugt größer 2, weiter bevorzugt größer 5, insbesondere bevorzugt größer 10 auf. Die Längserstreckung des Sensorgehäuses entspricht der Orientierung zwischen Befestigungsmittel und dem in dem zu messenden Medium liegenden Ende des Sensorgehäuses. Vorzugsweise verläuft die Eintauchrichtung, in welcher der Streulichtsensor in das zu messende Medium eingetaucht oder in die entsprechende Leitung oder den entsprechenden Behälter für das zu messende Medium eingeführt wird, parallel zur Längserstreckung des Sensorgehäuses.

Das Sensorgehäuse ist bevorzugt im Wesentlichen zylindrisch ausgebildet, insbesondere bevorzugt als Kreiszylinder, weiter bevorzugt als senkrechter Kreiszylinder. Im Bereich der Außenwand des Sensorgehäuses, in welchem Austrittfensterbereich und Eintrittsfensterbereich angeordnet sind, erfolgt in einer vorteilhaften Weiterbildung eine Abweichung von dem zylindrischen Aufbau. Ebenso liegt es im Rahmen der Erfindung, dass das Sensorgehäuse mehrere kreiszylindrische Teilbereich mit unterschiedlichem Durchmesser aufweist.

Der zylindrische Aufbau ermöglicht die Verwendung von handelsüblich erhältlichen Rohren als Grundmaterial für das Sensorgehäuse, sodass eine kostengünstige Herstellung ermöglicht wird.

Das Sensorgehäuse weist vorteilhafterweise an zumindest einer Seitenwand eine ebene Fläche auf, wobei innerhalb der ebenen Fläche der Austrittsfensterbereich und der Eintrittsfensterbereich angeordnet sind.

Eine solche ebene Fläche vereinfacht das gegenüber dem zu messenden Medium abgedichtete Einbringen von Austrittsfensterbereich und Eintrittsfensterbereich innerhalb der Wand des Sensorgehäuses.

Insbesondere ist es vorteilhaft, dass Eintrittsfensterbereich und Austrittsfensterbereich an der Außenseite eben ausgebildet sind, insbesondere mit planparallelen Innen- und Außenseiten ausgebildet sind. Hierdurch können Vor- und Rücksprünge an der Außenseite des Sensorgehäuses vermieden werden, sodass das Risiko von Schmutzablagerungen gesenkt wird.

Um eine Verfälschung des Messergebnisses durch unerwünschte Streustrahlungen, welche nicht an dem Medium reflektiert oder gesteuert wird, zu vermeiden, ist es vorteilhaft, dass zwischen Eintrittsfensterbereich und Austrittsfensterbereich ein für die Messstrahlung undurchlässiger Abschattungsbereich angeordnet ist. Hierdurch wird vermieden, dass Messstrahlung der Strahlungsquelle von dem Eintrittsfensterbereich unmittelbar, d. h. ohne Kontakt mit dem zu messenden Medium, in den Austrittsfensterbereich und von dort zu dem Detektor gelangt.

Insbesondere ist es vorteilhaft, dass Eintrittsfensterbereich und Austrittsfensterbereich in einem gemeinsamen, für die Messstrahlung durchlässigen Trägerelement ausgebildet sind, wobei zwischen Eintrittsfensterbereich und dem Austrittsfensterbereich der Abschattungsbereich in dem Trägerelement ausgebildet ist. Hierdurch ergibt sich der Vorteil, dass auf ein gemeinsames Element für Austrittsfensterbereich und Eintrittsfensterbereich zurückgegriffen werden kann.

In einer alternativen vorteilhaften Ausgestaltung sind Eintrittsfensterbereich und Austrittsfensterbereich als separate Elemente ausgebildet. Hierdurch ergibt sich der Vorteil einer erhöhten Stabilität. Insbesondere ist es vorteilhaft, dass Eintrittsfensterbereich und Austrittsfensterbereich als zwei separate Fenster ausgebildet sind und der Abschattungsbereich als ein zwischen den beiden Fenstern verlaufender Steg, insbesondere ein durch die Außenwand des Sensorgehäuses ausgebildeter Steg, ausgebildet ist. Es ist konstruktiv zum Erzielen einer hohen Robustheit insbesondere vorteilhaft, in dem Sensorgehäuse für den Eintrittsfensterbereich und den Austrittsfensterbereich jeweils einer Aussparung vorzusehen.

In einer vorteilhaften Weiterbildung ist der Streulichtsensor derart ausgebildet, dass mittels des Halterungselementes, insbesondere mittels des Halterungselementes und weitere Abschirmelemente der Detektor gegenüber indirekten Strahlengängen zwischen Strahlungsquelle und Detektor innerhalb des Sensorgehäuses abgeschirmt ist. Hierdurch ergibt sich der Vorteil, dass auch durch die Strahlungsquelle erzeugte Messstrahlung, welche innerhalb des Sensors durch Reflexion und/oder Brechung zu dem Detektor gelangen könnte und somit die Messergebnisse verfälschen könnte, abgeschirmt wird.

Vorteilhafterweise ist in dem Halterungselement für die Strahlungsquelle und für den Detektor jeweils eine Ausnehmung ausgebildet. Hierdurch wird eine robuste Anordnung von Detektor und Strahlungsquelle in dem Halterungselement ermöglicht. Insbesondere ist es vorteilhaft, dass die Ausnehmung umlaufend formschlüssig jeweils für den Detektor und die Strahlungsquelle ausgebildet ist, insbesondere umlaufend formschlüssig in einer Ebene senkrecht zur optischen Achse.

Vorteilhafterweise weist das Halterungselement einen Strahlungsschacht für den Detektor und einen Strahlungsschacht für die Strahlungsquelle auf. Das Halterungselement ist bevorzugt derart angeordnet, dass der Strahlungsschacht für den Detektor an dem Eintrittsfensterbereich und der Strahlungsschacht für die Strahlungsquelle an dem Austrittsfensterbereich endet. Die Strahlungsschächte sind bevorzugt zylindrisch, insbesondere bevorzugt Kreiszylindrisch ausgebildet. Hierdurch wird eine Abschattung gegenüber direkter Messstrahlung zwischen Strahlungsquelle und Detektor innerhalb des Sensorgehäuses erzielt.

Zwischen Detektor und Strahlungsquelle ist eine um das Halterungselement umlaufende Abschirmung, innerhalb des Sensorgehäuses angeordnet. Hierdurch wird vermieden, dass in einem gegebenenfalls durch technische Toleranzen bedingten Zwischenraum zwischen dem Halterungselement und der Innenwand des Sensorgehäuses und/oder dem Eintrittsfensterbereich und dem Austrittsfensterbereich Streustrahlung innerhalb des Sensorgehäuses von der Strahlungsquelle zu dem Detektor gelangt. Die Abschirmung ist bevorzugt aus einem flexiblen Material ausgebildet.

Das Halterungselement ist bevorzugt zumindest in einem Teilbereich formschlüssig, insbesondere in einer Ebene senkrecht zur Längserstreckung des Sensorgehäuses formschlüssig an der Innenwand des Sensorgehäuses anliegend ausgebildet.

Um ein Verdrehen des Halterungselementes innerhalb des Sensorgehäuses, insbesondere um eine zur Längserstreckung des Sensorgehäuses parallele Achse zu vermeiden, ist es vorteilhaft, dass das Sensorgehäuse ein Verdrehsicherungselement aufweist, an welchem das Halterungselement formschlüssig anliegend ausgebildet ist. Das Verdrehsicherungselement kann als Zapfen ausgebildet sein, sodass das Halterungselement eine entsprechende Ausnehmung für den Zapfen aufweist. Ebenso liegt es im Rahmen der Erfindung, dass das Halterungselement einen Zapfen und das Sensorgehäuse eine entsprechende Ausnehmung für den Zapfen aufweist. Eine besonders konstruktiv einfache Ausgestaltung ergibt sich, indem das Sensorgehäuse eine oder mehrere plane Ebenen aufweist, die nicht senkrecht zur Längserstreckung des Sensorgehäuses stehen und das Halterungselement derart ausgebildet ist, dass es formschlüssig an der oder den planen Ebenen anliegt.

Vorteilhafterweise ist in dem Halterungselement zumindest eine nicht-geradlinige Kabelführung ausgebildet, welche das Halterungselement in Richtung der Längserstreckung des Sensorgehäuses durchdringt.

Hierdurch kann in robuster und platzsparender Weise eine elektrische Kontaktierung des den Endbereich des Sensorgehäuses zugewandten Elementes aus der Gruppe Strahlungsquelle, Detektor, erfolgen.

Insbesondere ist es vorteilhaft, dass die Kabelführung an der dem Endbereich des Sensorgehäuses zugewandten Seite des Halterungselementes eine Eingangsöffnung aufweist, welche an einem dem Eintritts- und Austrittsfensterbereich abgewandten Bereich des Haltungselementes ausgebildet ist und an der dem Befestigungsbereich zugewandten Seite des Haltungselementes eine Ausgangsöffnung aufweist, welche an einem dem Eintritts- und Austrittsfensterbereich zugewandten Bereich des Halterungselement ausgebildet ist. Hierdurch kann in robuster und platzsparender Weise auf der dem Befestigungsmittel zugewandten Seite des Halterungselementes eine Kontaktierung des dort angeordneten Elementes aus der Gruppe Strahlungsquelle, Detektor an der dem Eintritts- und Austrittsfensterbereich abgewandten Seite des Halterungselementes erfolgen und in platzsparender Weise tritt das Kabel des anderen Elementes aus der Gruppe Strahlungsquelle, Detektor an der dem Eintrittsfensterbereich und Austrittsfensterbereich zugewandten Seite des Halterungselementes aus.

Untersuchungen der Anmelder haben ergeben, dass vorteilhafterweise Strahlungsquelle und Detektor derart angeordnet sind, dass sich die optischen Achsen von Strahlungsquelle und Detektor außerhalb des Sensorgehäuses mit einem Abstand zu dem Sensorgehäuse im Bereich 0,5 mm bis 5 mm schneiden. Hierdurch wird ein verbessertes Messergebnis erzielt.

Es liegt im Rahmen der Erfindung, dass das Halterungselement mehrteilig ausgebildet ist. Insbesondere liegt es im Rahmen der Erfindung, dass das Halterungselement ein Teilelement zur Aufnahme der Strahlungsquelle und ein weiteres Teilelement zur Aufnahme des Detektors aufweist, welche unter Zwischenschaltung weiterer Teilelemente oder bevorzugt direkt aneinanderliegend in dem Sensorgehäuse angeordnet werden.

Eine konstruktiv einfache Ausgestaltung und darüber hinaus robuste Ausgestaltung wird erzielt, indem in einer vorteilhaften Ausgestaltung das Halterungselement einteilig ausgebildet ist.

Die Messstrahlquelle kann in an sich bekannter Weise als Lampe oder Glühbirne ausgebildet sein. Für einen platzsparenden und robusten Aufbau ist es vorteilhaft, die Messstrahlquelle als Diode, insbesondere Leuchtdiode oder Laserdiode, auszubilden. Es liegt im Rahmen der Erfindung, die Messstrahlquelle zum Erzeugen eines Messstrahls im sichtbaren Bereich oder bevorzugt im Infrarotbereich, insbesondere im Bereich mit einer Wellenlänge größer 800 nm, bevorzugt im Bereich 800 nm bis 900 nm, auszubilden.

Der Detektor kann in an sich bekannter Weise als Strahlungsdetektor für die von der Strahlungsquelle erzeugte Messstrahlung ausgebildet sein. Insbesondere ist es vorteilhaft, den Detektor als Photodiode auszubilden.

Typische Strahlungsquellen zum Erzeugen eines Messstrahls erzeugen eine gerichtete Strahlung, sodass sich aus dieser Strahlungsrichtung die optische Achse der Strahlungsquelle ergibt. Bei Strahlungsquellen mit gleichförmiger Intensitätsverteilung der erzeugten Messstrahlung ergibt sich die optische Achse aus der mittig im Strahlungsfeld liegenden Strahlungsrichtung.

Ebenso weisen typische Detektoren eine bevorzugte Detektionsrichtung auf, aus welcher sich die optische Achse des Detektors ergibt. Bei heterogenen, für große Raumwinkel detektierende Detektoren ergibt sich die optische Achse aus der mittig liegenden Richtung im Detektionsraumwinkel des Detektors.

Das Halterungselement ist bevorzugt aus Kunststoff, insbesondere aus temperaturstabilem Kunststoff, ausgebildet. Bevorzugt ist das Halterungselement elektrisch nichtleitend ausgebildet. Der Streulichtsensor ist bevorzugt passend für pH-Einbauplätze ausgebildet. Hierdurch kann der Streulichtsensor in kostengünstiger Weise zur Trübungsmessung von Flüssigkeiten verwendet werden, bei welchen wahlweise auch eine Überwachung des pH-Wertes erfolgt.

Wie zuvor ausgeführt, sind Strahlungsquelle und Detektor derart an dem Halterungselement angeordnet, dass eine optische Achse der Strahlungsquelle mit einer optischen Achse des Detektors einen Winkel im Bereich von 50° bis 130° einschließt. Vorteilhafterweise liegt dieser Winkel im Bereich 70° bis 110°, insbesondere bevorzugt im Bereich 80° bis 100°, weiter bevorzugt im Bereich 85° bis 95°.

Der erfindungsgemäße Streulichtsensor weist den Vorteil auf, dass eine robuste und gleichzeitig schmale Ausbildung ermöglicht wird. Vorteilhafterweise ist der Streulichtsensor derart ausgebildet, dass ein den Streulichtsensor einhüllender Kreiszylinder einen Durchmesser kleiner 30 mm, bevorzugt kleiner 20 mm, insbesondere bevorzugt kleiner 15 mm aufweist.

Die Länge des Streulichtsensors ausgehend von den Befestigungsmittel zu dem bei Verwendung in Medium liegenden Ende des Sensorgehäuses ist bevorzugt größer 10 cm, insbesondere bevorzugt größer 12 cm.

Das Befestigungsmittel ist bevorzugt passend für eine PG-13/5-Verschraubung ausgebildet. Hierdurch wird der Einsatz an typischen, für pH-Sensoren vorgesehenen Messstellen ermöglicht.

Vorteilhafterweise wird die Strahlungsquelle derart in dem Halterungselement angeordnet, dass die optische Achse der Strahlungsquelle mit der Längserstreckung des Sensorgehäuses einen Winkel im Bereich 40° bis 50°, bevorzugt 42° bis 47°, einschließt. Ebenso ist es vorteilhaft, dass der Detektor derart an dem Halterungselement angeordnet ist, dass die optische Achse des Detektors mit der Längserstreckung des Sensorgehäuses einen Winkel im Bereich 40° bis 50°, insbesondere bevorzugt 42° bis 47°, einschließt.

Vorteilhafterweise weicht der Winkel, den die optische Achse der Strahlungsquelle mit der Längserstreckung des Sensorgehäuses einschließt, um weniger als 15°, bevorzugt um weniger als 10°, insbesondere bevorzugt um weniger als 5° von dem Winkel ab, den die optische Achse des Detektors mit der Längserstreckung des Sensorgehäuses einschließt.

Die eingangs genannte Aufgabe ist weiterhin durch ein Verfahren zur Herstellung des Streulichtsensors gemäß Anspruch 13 gelöst. Das Verfahren weist folgende Verfahrensschritte auf:
A. Anordnen einer Strahlungsquelle und eines Detektors in einem Halterungselement und Anordnen einer um das Halterungselement (10) umlaufende Abschirmung (14) zwischen Detektor (2) und Strahlungsquelle (1);
B. Anordnen eines Austrittsfensterbereichs und eines Eintrittsfensterbereichs in einer Seitenwand eines länglichen Sensorgehäuses;
C. Einbringen des Halterungselementes in das Sensorgehäuse, sodass eine optische Achse der Strahlungsquelle den Austrittsfensterbereich und eine optische Achse des Detektors den Eintrittsfensterbereich durchdringt.

Hierdurch wird ein kostengünstiges Herstellungsverfahren für einen kompakten und dennoch robusten Rückstreusensor erzielt.

Vorteilhafterweise wird vor Verfahrensschritt C in dem Sensorgehäuse eine Montageöffnung ausgebildet, insbesondere in einer dem Eintrittsfensterbereich und/oder dem Austrittsfensterbereich gegenüberliegenden Seitenwand des Sensorgehäuses und nach Verfahrensschritt C wird die Montageöffnung verschlossen. Hierdurch ergibt sich der Vorteil eines unaufwendigen Einsetzens und Justierens des Halterungselements in das Sensorgehäuse.

Die um das Halterungselement umlaufende Abschirmung zwischen Detektor und Strahlungsquelle welche innerhalb des Sensorgehäuses angeordnet wird, ist bevorzugt aus einem flexiblen Material ausgebildet.

Weitere vorteilhafte Merkmale und Ausgestaltungen werden im Folgenden anhand eines Ausführungsbeispiels und den Figuren erläutert. Dabei zeigt:
- Figur 1: eine Schnittdarstellung eines Ausführungsbeispiel eines erfindungsgemäßen Streulichtsensors,
- Figur 2: eine Schnittdarstellung gemäß Schnittebene A in Figur 1 und
- Figur 3: eine Ausschnittsvergrößerung B aus Figur 1.

Sämtliche Figuren zeigen schematische, nicht maßstabsgetreue Darstellungen. Gleiche Bezugszeichen in den Figuren bezeichnen gleich oder gleichwirkende Elemente.

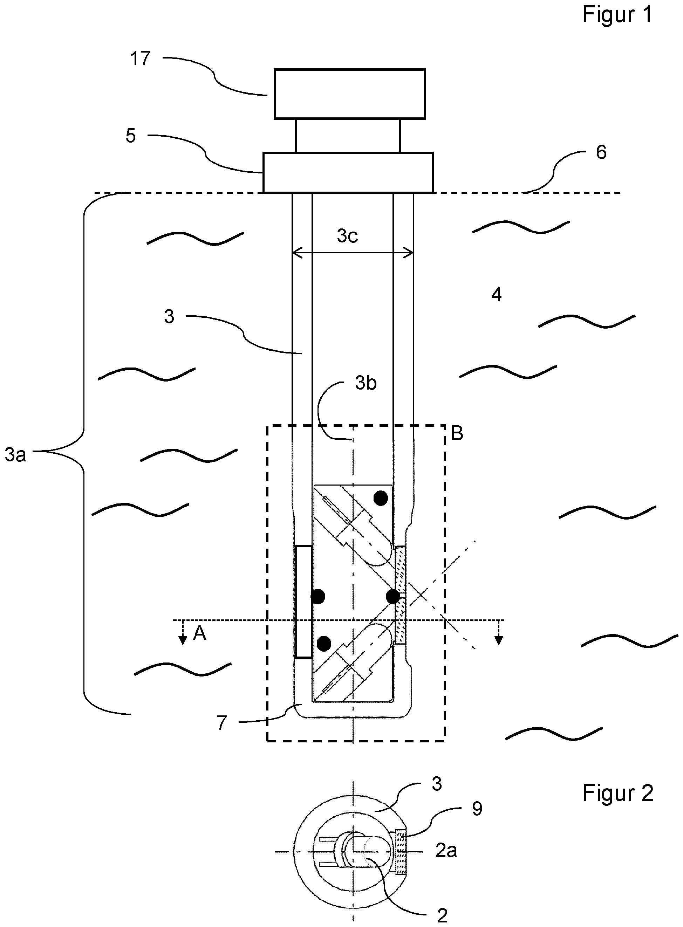

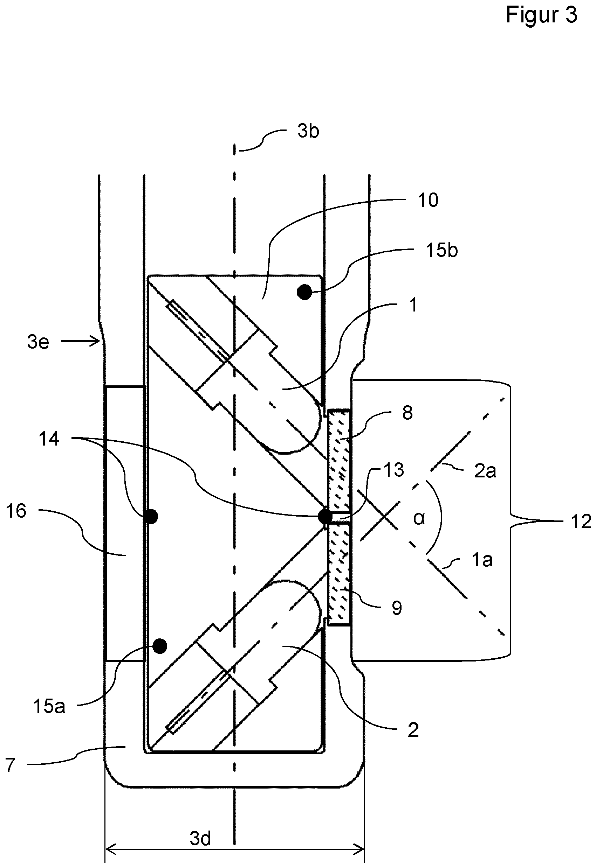

Figur 1 zeigt ein Ausführungsbeispiel eines erfindungsgemäßen Streulichtsensors zur Trübungsmessung, vorliegend zur Trübungsmessung in Wasserkreisläufen. Das im Kreislauf aufbereitete Wasser stellt somit das zu messende Medium dar. Zur besseren Darstellbarkeit der Details und Bezugszeichen ist in Figur 3 eine Ausschnittsvergrößerung des in Figur 1 mit B gekennzeichneten Bereichs gezeigt.

Der Streulichtsensor weist eine Strahlungsquelle 1 auf, welche vorliegend als Leuchtdiode zum Erzeugen eines Messstrahls mit einer Wellenlänge von 850 nm ausgebildet ist. Der Messstrahl wird entlang der optischen Achse 1a ausgesandt. Der Rückstreusensor weist weiterhin einen Detektor 2 zum Detektieren des in dem vorliegend als Wasser ausgebildetem Medium 4 zumindest teilweise reflektierten und/oder gestreuten Messstrahls als Empfangsstrahl. Der Detektor 2 ist als Photodiode ausgebildet und weist eine Vorzugsrichtung für zu detektierende einfallende Strahlung auf, welche die optische Achse 2a des Detektors definiert.

Der Streulichtsensor weist weiterhin ein längliches Sensorgehäuse 3 auf zum Eintauchen in das Medium 4. Die Strahlungsquelle 1 und der Detektor 2 sind in dem Sensorgehäuse 3 angeordnet. Der Streulichtsensor weist weiterhin ein Befestigungsmittel 5 auf, an welchem das Sensorgehäuse 3 angeordnet ist. Das Befestigungsmittel ist zum Anordnen des Streulichtsensors an eine Behälterwand 6 ausgebildet. Vorliegend ist das Befestigungsmittel 5 als PG-13,5-Verschraubung ausgebildet. Der Behälter stellt vorliegend ein Leitungsrohr im Leitungssystem eines Wasserkreislaufs dar und an einer Wand 6 des Leitungsrohrs ist ein entsprechendes Gegenstück zur PG-13/5-Verschraubung ausgebildet, um den Streulichtsensor mittels des Befestigungsmittels 5 an der Behälterwand 6 zu befestigen, wobei das Sensorgehäuse 3 in den Behälter, vorliegend in das Leitungsrohr, hineinragt und somit mit dem Medium 4 in Kontakt steht.

Das Sensorgehäuse ist länglich ausgebildet und weist eine Länge 3a von 12 cm auf. Das Sensorgehäuse weist im Wesentlichen die Form eines zweistufigen Kreiszylinders auf, mit einem Durchmesser 3c in einem oberen Bereich des Sensorgehäuses von 12 mm und in einem unteren Bereich des Sensorgehäuses einen Durchmesser 3d von 11 mm. Die Durchmesserreduzierung erfolgt an einer Stufe 3e.

An einer seitlichen Wand des Sensorgehäuses zwischen Befestigungsmittel 5 und einem Endbereich 7 des Sensorgehäuses 3 ist ein Austrittsfensterbereich 8 für den Austritt des Messstrahls aus dem Sensorgehäuse 3 und ein Eintrittsfensterbereich 9 für den Eintritt des Empfangsstrahls in das Sensorgehäuse ausgebildet.

Der Streulichtsensor weist ein in dem Sensorgehäuse 3 angeordnetes Halterungselement 10 auf, an welchem Strahlungsquelle 1 und Detektor 2 derart angeordnet sind, dass die optische Achse 1a der Strahlungsquelle 1 mit der optischen Achse 2a der Strahlungsquelle 2 einen Winkel α im Bereich 50° bis 130°, vorliegend 90°, einschließt.

Das Halterungselement 10 ist als optisches Abschattungselement für die Messstrahlung der Strahlungsquelle 1 ausgebildet, sodass der Detektor zwei gegenüber direkten Strahlengängen (siehe beispielsweise Strahlengang 11, gestrichelte Linie) zwischen Strahlungsquelle 1 und Detektor 2 innerhalb des Sensorgehäuses 3 abgeschattet ist.

Austrittsfensterbereich 8 und Eintrittsfensterbereich 9 sind parallel zu einer Längserstreckung 3b des länglichen Sensorgehäuses 3 angeordnet.

Das Sensorgehäuse weist an der Seitenwand, an welcher Eintrittsfensterbereich und Austrittsfensterbereich 8 angeordnet sind, eine ebene Fläche 12 auf. Die ebene Fläche 12 steht in der Darstellung gemäß Figur 1 senkrecht zur Zeichenebene. Innerhalb der ebenen Fläche sind Austrittsfensterbereich und Eintrittsfensterbereich angeordnet.

Der Eintrittsfensterbereich 9 und der Austrittsfensterbereich 8 sind als zwei separate Fenster ausgebildet. Zwischen Eintrittsfensterbereich 9 und Austrittsfensterbereich 8 ist ein durch die seitliche Wand des Sensorgehäuses 3, welche als ebene Fläche 12 ausgebildet ist, ausgebildeter Steg 13 angeordnet, welcher undurchlässig für die Messstrahlung der Strahlungsquelle 1 ist. Der Steg 13 bildet somit einen Abschattungsbereich, welcher Strahlengänge zwischen Austrittsfensterbereich 8 und Eintrittsfensterbereich 9 unterbindet.

Zwischen Detektor 2 und Strahlungsquelle 1 ist eine um das Halterungselement 10 umlaufende Abschirmung 14, welche vorliegend als O-Ring ausgebildet ist, innerhalb des Sensorgehäuses 3 angeordnet. Die umlaufende Abschirmung 17 liegt somit an der Innenseite an dem Halterungselement 10 und an der Außenseite an der Innenwand des Sensorgehäuses 3 an, insbesondere teilweise an der Innenseite des Stegs 13.

Das Halterungselement 10 ist formschlüssig an der Innenwand des Sensorgehäuses 3 anliegend ausgebildet, in Teilbereichen oberhalb und unterhalb der umlaufenden Abschirmung 14.

Das Halterungselement 10 weist eine als Kabelkanal ausgebildete Kabelführung auf, vorliegend für das Anschlusskabel des Detektors 2. Die Kabelführung weist an der dem Endbereich 7 des Sensorgehäuses zugewandten Seite des Halterungselementes 10 eine Eingangsöffnung 15a auf, welche an einem den Eintritts- und Austrittsfensterbereich (8, 9) abgewandten Bereich des Halterungselements 10 ausgebildet ist und an der dem Befestigungsmittel 5 zugewandten Seite des Halterungselements 10 eine Ausgangsöffnung 15b, welche an einem dem Eintritts- und Austrittsfensterbereich (8, 9) zugewandten Bereich des Halterungselementes 10 ausgebildet ist.

Strahlungsquelle 1 und Detektor 2 sind derart angeordnet, dass sich die optischen Achsen von Strahlungsquelle und Detektor (1a, 2a) außerhalb des Sensorgehäuses 3 mit einem Abstand zum Sensorgehäuse im Bereich von 0,5 mm bis 5 mm, vorliegend 3 mm, schneiden.

Zur Herstellung des in Figur 1 dargestellten Streulichtsensors werden Strahlungsquelle 1 und Detektor 2 in dem Halterungselement 10 angeordnet. Austrittsfensterbereich 8 und der Eintrittsfensterbereich 9 werden in entsprechende Ausnehmungen des Sensorgehäuses 3 eingesetzt. Durch die Montageöffnung 16 erfolgt ein Reinigen der Innenseite von Austrittsfensterbereich 8 und der Eintrittsfensterbereich 9.

Das Halterungselement 10 wird mit Strahlungsquelle 1 und Detektor 2 in dem Sensorgehäuse eingebracht, sodass die optische Achse 1a der Strahlungsquelle 1 den Austrittsfensterbereich 8 und die optische Achse 2a des Detektors 2 den Eintrittsfensterbereich 9 durchdringt.

Anschließend wird die Montageöffnung 16 verschlossen.

Strahlungsquelle 1 und Detektor 2 sind über elektrische Leitungen mit einer Auswerteeinheit 17 verbunden, um die Strahlungsquelle 1 mit elektrischer Energie zu versorgen und die Messsignale des Detektors 2 auszulesen und zu verarbeiten, insbesondere die analogen Signale des Detektors 2 in digitale Signale zu wandeln.

Figur 2 zeigt eine Schnittdarstellung gemäß Schnittebene A in Figur 1, welche in der Darstellung gemäß Figur 1 senkrecht zur Zeichenebene steht. Aus Gründen der besseren Darstellerbarkeit wird das Halterungselement 10 nicht gezeigt.

### Bezugszeichenliste

- 1: Strahlungsquelle
- 1a: optische Achse der Strahlungsquelle
- 2: Detektor
- 2a: optische Achse des Detektors
- 3: Sensorgehäuse
- 3a: Länge des Sensorgehäuses
- 3b: Längserstreckung
- 3c: Durchmesser
- 3d: Durchmesser
- 3e: Stufe
- 4: Medium
- 5: Befestigungsmittel
- 6: Behälterwand
- 7: Endbereich
- 8: Austrittsfensterbereich
- 9: Eintrittsfensterbereich
- 10: Halterungselement
- 11: direkter Strahlengang
- 12: ebene Fläche
- 13: Steg
- 14: umlaufende Abschirmung
- 15a: Eingangsöffnung
- 15b: Ausgangsöffnung
- 16: Montageöffnung
- 17: Auswerteeinheit

## Patentansprüche

1. Streulichtsensor zur Trübungsmessung in einem flüssigen, pastösen, pulvrigen und/oder gasförmigen Medium (4),
mit einer Strahlungsquelle (1) zum Erzeugen eines Messstrahls,
einem Detektor (2) zum Detektieren des in dem Medium (4) zumindest teilweise reflektierten und/oder gestreuten Messstrahls als Empfangsstrahl, einem länglichen Sensorgehäuse zum Eintauchen in das Medium (4), wobei die Strahlungsquelle (1) und der Detektor (2) in dem Sensorgehäuse (3) angeordnet sind,
und mit einem Befestigungsmittel zum Anordnen des Streulichtsensors an einer Behälterwand (6),
wobei in einer seitlichen Wand des Sensorgehäuses zwischen Befestigungsmittel (5) und einem Endbereich (7) des Sensorgehäuses zumindest ein Austrittsfensterbereich (8) für den Austritt des Messstrahls aus dem Sensorgehäuse (3) und ein Eintrittsfensterbereich (9) für den Eintritt des Empfangsstrahls in das Sensorgehäuse (3) ausgebildet ist,
der Streulichtsensor ein in dem Sensorgehäuse (3) angeordnetes Halterungselement (10) aufweist, an welchem Strahlungsquelle (1) und Detektor (2) derart angeordnet sind, dass eine optische Achse der Strahlungsquelle (1a) mit einer optischen Achse des Detektors (2a) einen Winkel im Bereich 50° bis 130° einschließt, das Halterungselement (10) als optisches Abschattungselement für den Messstrahl ausgebildet ist, sodass der Detektor (2) gegenüber direkten Strahlengängen zwischen Strahlungsquelle (1) und Detektor (2) innerhalb des Sensorgehäuses abgeschattet ist, und
zwischen Detektor (2) und Strahlungsquelle (1) eine um das Halterungselement (10) umlaufende Abschirmung (14) innerhalb des Sensorgehäuses angeordnet ist.

2. Streulichtsensor nach Anspruch 1, wobei der Austrittsfensterbereich (8) und der Eintrittsfensterbereich (9) parallel zu einer Längserstreckung (3b) des länglichen Sensorgehäuses angeordnet sind.

3. Streulichtsensor nach einem der vorangegangenen Ansprüche, wobei das Sensorgehäuse im Wesentlichen zylindrisch ausgebildet ist.

4. Streulichtsensor nach einem der vorangegangenen Ansprüche, wobei das Sensorgehäuse (3) an zumindest einer Seitenwand eine ebene Fläche (12) aufweist und innerhalb der ebenen Fläche der Austrittsfensterbereich (8) und der Eintrittsfensterbereich (9) angeordnet sind.

5. Streulichtsensor nach einem der vorangegangenen Ansprüche,
wobei zwischen Eintrittsfensterbereich (9) und Austrittsfensterbereich (8) ein für die Messstrahlung undurchlässiger Abschattungsbereich angeordnet ist,

6. Streulichtsensor nach Anspruch 5,
wobei Eintrittsfensterbereich (9) und Austrittsfensterbereich (8) in einem gemeinsamen für die Messstrahlung durchlässigen Trägerelement ausgebildet sind, wobei zwischen Eintrittsfensterbereich (9) und Austrittsfensterbereich (8) der Abschattungsbereich in dem Trägerelement ausgebildet ist oder Eintrittsfensterbereich (9) und Austrittsfensterbereich (8) als zwei separate Fenster ausgebildet sind und der Abschattungsbereich als ein zwischen den beiden Fenstern verlaufender Steg (13) ausgebildet ist.

7. Streulichtsensor nach einem der vorangegangenen Ansprüche, wobei mittels des Halterungselementes, insbesondere mittels des Halterungselementes und weiterer Abschirmungselemente der Detektor (2) gegenüber indirekten Strahlengängen zwischen Strahlungsquelle (1) und Detektor (2) innerhalb des Sensorgehäuses abgeschirmt ist.

8. Streulichtsensor nach einem der vorangegangenen Ansprüche,
wobei in dem Halterungselement (10) für die Strahlungsquelle (1) und für den Detektor (2) jeweils eine Ausnehmung ausgebildet ist, insbesondere,
wobei in dem Halterungselement (10) ein Strahlungsschacht für den von der Strahlungsquelle (1) erzeugten Messstrahl zwischen Strahlungsquelle (1) und Austrittsfensterbereich (8) und ein Strahlungsschacht für den durch den Detektor (2) detektierten Empfangsstrahl zwischen Detektor (2) und Eintrittsfensterbereich (9) ausgebildet ist.

9. Streulichtsensor nach einem der vorangegangenen Ansprüche, wobei das Halterungselement (10) zumindest in einem Teilbereich formschlüssig, insbesondere in einer Ebene senkrecht zur Längserstreckung (3b) des Sensorgehäuses formschlüssig an der Innenwand des Sensorgehäuses anliegend ausgebildet ist.

10. Streulichtsensor nach einem der vorangegangenen Ansprüche, wobei in dem Halterungselement (10) zumindest eine nicht-geradlinige Kabelführung ausgebildet ist, welche das Halterungselement (10) in Richtung der Längserstreckung (3b) des Sensorgehäuses durchdringt.

11. Streulichtsensor nach Anspruch 10, wobei die Kabelführung an der dem Endbereich (7) des Sensorgehäuses zugewandten Seite des Halterungselements eine Eingangsöffnung (15a) aufweist, welche an einem dem Eintritts- und Austrittsfensterbereich (8, 9) abgewandten Bereich des Halterungselements ausgebildet ist und an der dem Befestigungsmittel (5) zugewandten Seite des Halterungselements eine Ausgangsöffnung (15b) aufweist, welche an einem dem Eintritts- und Austrittsfensterbereich (8, 9) zugewandten Bereich des Halterungselements ausgebildet ist.

12. Streulichtsensor nach einem der vorangegangenen Ansprüche, wobei Strahlungsquelle (1) und Detektor (2) derart angeordnet sind, dass sich die optischen Achsen von Strahlungsquelle (1) und Detektor (2) außerhalb des Sensorgehäuses mit einem Abstand zum Sensorgehäuse (3) im Bereich 0,5 mm bis 5 mm schneiden.

13. Verfahren zur Herstellung eines Streulichtsensors nach einem der vorangegangenen Ansprüche, mit den Verfahrensschritten
A. Anordnen einer Strahlungsquelle (1) und eines Detektors in einem Halterungselement und Anordnen einer um das Halterungselement (10) umlaufenden Abschirmung (14) zwischen Detektor (2) und Strahlungsquelle (1);
B. Anordnen eines Austrittsfensterbereichs und eines Eintrittsfensterbereichs in einer Seitenwand eines länglichen Sensorgehäuses;
C. Einbringen des Halterungselementes in das Sensorgehäuse (3), sodass eine optische Achse der Strahlungsquelle (1a) den Austrittsfensterbereich (8) und eine optische Achse des Detektors (2a) den Eintrittsfensterbereich (9) durchdringt.

14. Verfahren nach Anspruch 13, wobei vor Verfahrensschritt C in dem Sensorgehäuse (3) eine Montageöffnung (16) ausgebildet wird, insbesondere an einer dem Eintrittsfensterbereich (9) und/oder dem Austrittsfensterbereich (8) gegenüberliegenden Seitenwand des Sensorgehäuses, und nach Verfahrensschritt C die Montageöffnung (16) verschlossen wird.

## Claims

1. Scattered light sensor for measuring turbidity in a liquid, pasty, powdery and/or gaseous medium (4),
comprising a radiation source (1) for generating a measuring beam,
a detector (2) for detecting the measuring beam at least partially reflected and/or scattered in the medium (4) as a receiving beam,
an elongate sensor housing for immersion in the medium (4), wherein the radiation source (1) and the detector (2) are arranged in the sensor housing (3), and comprising a fastening means for arranging the scattered light sensor on a container wall (6),
wherein in a side wall of the sensor housing between the fastening means (5) and an end region (7) of the sensor housing at least one exit window region (8) for the exit of the measuring beam from the sensor housing (3) and an entry window region (9) for the entry of the receiving beam into the sensor housing (3) are formed,
the scattered light sensor has a holding element (10) arranged in the sensor housing (3), on which the radiation source (1) and detector (2) are arranged such that an optical axis of the radiation source (1a) encloses an angle in the range of 50° to 130° with an optical axis of the detector (2a),
the holding element (10) is designed as an optical shading element for the measuring beam, so that the detector (2) is shaded from direct beam paths between the radiation source (1) and the detector (2) within the sensor housing, and a shield (14) surrounding the holding element (10) is arranged within the sensor housing between the detector (2) and the radiation source (1).

2. Scattered light sensor according to claim 1,
wherein the exit window region (8) and the entry window region (9) are arranged parallel to a longitudinal extension (3b) of the elongate sensor housing.

3. Scattered light sensor according to either of the preceding claims,
wherein the sensor housing is substantially cylindrical.

4. Scattered light sensor according to any of the preceding claims,
wherein the sensor housing (3) has a flat surface (12) on at least one side wall and the exit window region (8) and the entry window region (9) are arranged within the flat surface.

5. Scattered light sensor according to any of the preceding claims,
wherein a shading region which is impermeable to the measuring radiation is arranged between the entry window region (9) and the exit window region (8).

6. Scattered light sensor according to claim 5,
wherein the entry window region (9) and the exit window region (8) are formed in a common carrier element which is permeable to the measuring radiation, wherein the shading region is formed in the carrier element between the entry window region (9) and the exit window region (8) or the entry window region (9) and the exit window region (8) are formed as two separate windows and the shading region is formed as a connecting piece (13) extending between the two windows.

7. Scattered light sensor according to any of the preceding claims,
wherein by means of the holding element, in particular by means of the holding element and further shielding elements, the detector (2) is shielded against indirect beam paths between the radiation source (1) and the detector (2) within the sensor housing.

8. Scattered light sensor according to any of the preceding claims,
wherein a recess is formed in the holding element (10) for the radiation source (1) and for the detector (2), in particular,
wherein in the holding element (10) a radiation shaft for the measuring beam generated by the radiation source (1) is formed between the radiation source (1) and the exit window region (8) and a radiation shaft for the receiving beam detected by the detector (2) is formed between the detector (2) and the entry window region (9).

9. Scattered light sensor according to any of the preceding claims,
wherein the holding element (10) is designed to be form-fitting at least in a partial region, in particular in a plane perpendicular to the longitudinal extension (3b) of the sensor housing, is designed to form-fittingly rest against the inner wall of the sensor housing.

10. Scattered light sensor according to any of the preceding claims,
wherein at least one non-rectilinear cable guide is formed in the holding element (10), which guide penetrates the holding element (10) in the direction of the longitudinal extension (3b) of the sensor housing.

11. Scattered light sensor according to claim 10,
wherein the cable guide has an inlet opening (15a) on the side of the holding element facing the end region (7) of the sensor housing, which opening is formed in a region of the holding element facing away from the entry and exit window region (8, 9), and has an outlet opening (15b) on the side of the holding element facing the fastening means (5), which opening is formed in a region of the holding element facing the entry and exit window region (8, 9).

12. Scattered light sensor according to any of the preceding claims,
wherein the radiation source (1) and the detector (2) are arranged such that the optical axes of the radiation source (1) and the detector (2) intersect outside the sensor housing at a distance from the sensor housing (3) in the range 0.5 mm to 5 mm.

13. Method for producing a scattered light sensor according to any of the preceding claims,
comprising the following method steps
A. arranging a radiation source (1) and a detector in a holding element and arranging a shield (14) surrounding the holding element (10) between the detector (2) and the radiation source (1);
B. arranging an exit window region and an entry window region in a side wall of an elongate sensor housing;
C. inserting the holding element into the sensor housing (3) so that an optical axis of the radiation source (1a) penetrates the exit window region (8) and an optical axis of the detector (2a) penetrates the entry window region (9).

14. Method according to claim 13, wherein
before method step C, a mounting opening (16) is formed in the sensor housing (3), in particular in a side wall of the sensor housing opposite the entry window region (9) and/or the exit window region (8), and after method step C, the mounting opening (16) is closed.

## Revendications

1. Capteur de lumière diffusée pour mesurer la turbidité dans un milieu (4) liquide, pâteux, pulvérulent et/ou gazeux, comprenant une source de rayonnement (1) pour générer un faisceau de mesure, un détecteur (2) pour détecter le faisceau de mesure au moins partiellement réfléchi et/ou dispersé dans le milieu (4) en tant que faisceau de réception, un boîtier de capteur oblong à immerger dans le milieu (4), dans lequel la source de rayonnement (1) et le détecteur (2) sont agencés dans le boîtier de capteur (3), et comprenant un moyen de fixation pour agencer le capteur de lumière diffusée sur une paroi de récipient (6), dans lequel au moins une zone de fenêtre de sortie (8) pour que le faisceau de mesure sorte du boîtier de capteur (3) et une zone de fenêtre d'entrée (9) pour que le faisceau de réception entre dans le boîtier de capteur (3) sont réalisées dans une paroi latérale du boîtier de capteur, entre le moyen de fixation (5) et une zone d'extrémité (7) du boîtier de capteur, le capteur de lumière diffusée présente un élément de support (10) agencé dans le boîtier de capteur (3), en lequel la source de rayonnement (1) et le détecteur (2) sont agencés de manière telle qu'un axe optique de la source de rayonnement (1a) forme avec un axe optique du détecteur (2a) un angle dans la plage de 50° à 130°, l'élément de support (10) est réalisé en tant qu'élément occultation optique pour le faisceau de mesure, de telle sorte que le détecteur (2) soit occulté par rapport aux trajectoires de faisceau directes entre la source de rayonnement (1) et le détecteur (2) à l'intérieur du boîtier de capteur, et un blindage (14) tournant autour de l'élément de support (10) est agencé à l'intérieur du boîtier de capteur, entre le détecteur (2) et la source de rayonnement (1).

2. Capteur de lumière diffusée selon la revendication 1, dans lequel la zone de fenêtre de sortie (8) et la zone de fenêtre d'entrée (9) sont agencées parallèlement à une étendue longitudinale (3b) du boîtier de capteur oblong.

3. Capteur de lumière diffusée selon l'une des revendications précédentes, dans lequel le boîtier de capteur est réalisé de manière sensiblement cylindrique.

4. Capteur de lumière diffusée selon l'une des revendications précédentes, dans lequel le boîtier de capteur (3) présente une surface plane (12) en au moins une paroi latérale et la zone de fenêtre de sortie (8) et la zone de fenêtre d'entrée (9) sont agencées à l'intérieur de la surface plane.

5. Capteur de lumière diffusée selon l'une des revendications précédentes, dans lequel une zone d'occultation imperméable au rayonnement de mesure est agencée entre la zone de fenêtre d'entrée (9) et la zone de fenêtre de sortie (8),

6. Capteur de lumière diffusée selon la revendication 5, dans lequel la zone de fenêtre d'entrée (9) et la zone de fenêtre de sortie (8) sont réalisées dans un élément porteur commun perméable au rayonnement de mesure, dans lequel la zone d'occultation entre la zone de fenêtre d'entrée (9) et la zone de fenêtre de sortie (8) est réalisée dans l'élément porteur ou la zone de fenêtre d'entrée (9) et la zone de fenêtre de sortie (8) sont conformées en deux fenêtres séparées et la zone d'occultation est conformée en une nervure (13) s'étendant entre les deux fenêtres.

7. Capteur de lumière diffusée selon l'une des revendications précédentes, dans lequel le détecteur (2) est blindé par rapport aux trajectoires de faisceau indirectes entre la source de rayonnement (1) et le détecteur (2) à l'intérieur du boîtier de capteur au moyen de l'élément de support, en particulier au moyen de l'élément de support et d'autres éléments de blindage.

8. Capteur de lumière diffusée selon l'une des revendications précédentes, dans lequel un évidement respectif est réalisé dans l'élément de support (10) pour la source de rayonnement (1) et pour le détecteur (2), en particulier dans lequel, dans l'élément de support (10), un puits de rayonnement pour le faisceau de mesure généré par la source de rayonnement (1) est réalisé entre la source de rayonnement (1) et la zone de fenêtre de sortie (8) et un puits de rayonnement pour le faisceau de réception détecté par le détecteur (2) est réalisé entre le détecteur (2) et la zone de fenêtre d'entrée (9).

9. Capteur de lumière diffusée selon l'une des revendications précédentes, dans lequel l'élément de support (10) est réalisé au moins dans une zone partielle par complémentarité de forme, en particulier dans un plan perpendiculaire à l'étendue longitudinale (3b) du boîtier de capteur par complémentarité de forme en reposant contre la paroi intérieure du boîtier de capteur.

10. Capteur de lumière diffusée selon l'une des revendications précédentes, dans lequel dans l'élément de support (10) est réalisé au moins un câblage non rectiligne qui pénètre l'élément de support (10) en direction de l'étendue longitudinale (3b) du boîtier de capteur.

11. Capteur de lumière diffusée selon la revendication 10, dans lequel le câblage présente du côté tourné vers la zone d'extrémité (7) du boîtier de capteur de l'élément de support une ouverture d'entrée (15a) qui est réalisée en une zone éloignée de la zone de fenêtre d'entrée et de sortie (8, 9) de l'élément de support, et présente du côté tourné vers le moyen de fixation (5) de l'élément de support une ouverture de sortie (15b) qui est réalisée en une zone tournée vers la zone de fenêtre d'entrée et de sortie (8, 9) de l'élément de support.

12. Capteur de lumière diffusée selon l'une des revendications précédentes, dans lequel la source de rayonnement (1) et le détecteur (2) sont agencés de manière telle que les axes optiques de la source de rayonnement (1) et du détecteur (2) se croisent en dehors du boîtier de capteur, à une distance par rapport au boîtier de capteur (3) dans la plage de 0,5 mm à 5 mm.

13. Procédé de fabrication d'un capteur de lumière diffusée selon l'une des revendications précédentes, comprenant les étapes de procédé suivantes
A. agencer une source de rayonnement (1) et un détecteur dans un élément de support et agencer un blindage (14) tournant autour de l'élément de support (10) entre le détecteur (2) et la source de rayonnement (1) ;
B. agencer une zone de fenêtre de sortie et une zone de fenêtre d'entrée dans une paroi latérale d'un boîtier de capteur oblong ;
C. introduire l'élément de support dans le boîtier de capteur (3) de telle sorte qu'un axe optique de la source de rayonnement (1a) pénètre la zone de fenêtre de sortie (8) et un axe optique du détecteur (2a) pénètre la zone de fenêtre d'entrée (9).

14. Procédé selon la revendication 13, dans lequel avant l'étape de procédé C, une ouverture de montage (16) est réalisée dans le boîtier de capteur (3), en particulier en une paroi latérale opposée à la zone de fenêtre d'entrée (9) et/ou la zone de fenêtre de sortie (8) du boîtier de capteur, et après l'étape de procédé C, l'ouverture de montage (16) est fermée.
